# EUROPEAN PATENT SPECIFICATION

(11) **EP 3 116 553 B1**
(45) Date of publication and mention of the grant of the patent: **28.09.2022**
(21) Application number: 15712814.1
(22) Date of filing: 09.03.2015
(51) Int. Cl.: A61L 2/20, A61L 2/26

(54) **DISINFECTION APPARATUS**
DESINFEKTIONSGERÄT
APPAREIL DE DÉSINFECTION

(30) Priority: 09.03.2014 GB 201404127; 13.03.2014 GB 201404517
(43) Date of publication of application: 18.01.2017
(73) Proprietor: Lynch, Edward, Baggington CV8 3LA (GB)
(72) Inventor: BEDI, Raman, Potters Bar Hertfordshire EN6 1EL (GB)
(74) Representative: Ellis, Michael James
(86) International application number: PCT/EP2015/054867
(87) International publication number: WO 2015/135887

(56) References cited:
- WO-A2-03/068274
- US-A- 5 443 801
- US-A1- 2005 147 527
- US-A1- 2008 236 621
- US-B1- 6 620 379

## Description

### FIELD OF THE INVENTION

The present invention relates to the field of disinfection and sterilisation and in particular to the disinfection and/or sterilisation of products, apparatus and instruments, such as surgical or dental equipment, apparatus or tools and to a method of disinfection thereof and further relates to the field of topical therapeutic disinfective treatment of a patient and apparatus therefore.

### BACKGROUND OF THE INVENTION

It is necessary in order to reduce the risk and prevalence of infection resulting from dental or surgical intervention to ensure that dental and surgical instruments are clean and free from bacteria.

Common practice in dental and medical surgeries is to sterilise or disinfect dental or surgical tools prior to use by use of an autoclave. Autoclaves, however, are substantial and expensive pieces of equipment requiring an electrical supply and a water supply. Furthermore, whilst they are understood to effectively kill bacteria and other such microorganisms, they are believed to be less effective at dealing with prions and other proteinacious infective agents. Thus, a risk of infection with tainted surgical and dental equipment remains.

High level chemical disinfection (e.g. using chlorine or hypochlorite) is also used, as is heat treatment.

Ozone is a known disinfectant. It is used as a disinfectant of water supplies and ozonated water is used as a disinfecting fluid. Ozone gas is also used in gaseous ozone disinfection. The use of ozone gas as a disinfecting agent must be carried out with care and with suitable recovery (scavenging) of ozone molecules to prevent its release due to the toxicity of ozone.

CN 202844173U describes a device for generating and delivery ozonated water for disinfection of dental instruments chairside.

WO-A-2011/038487 illustrates a system and method for sterilising dental and surgical articles by sequential supply to an integral sterilisation chamber of hydrogen peroxide and ozone.

In dentistry, ozone gas can be used to disinfect tooth surfaces prior to treatment or to treat caries-producing bacterial in decaying tooth matter. A device for controlled delivery and capture of gaseous ozone to the tooth area may be used. An example of such a device is the Healozone^{™} marketed by Curozone Gmbh. This is described, for example, in WO-A-02/065936. US5443801 and WO03/068274 disclose an apparatus for sterilization of objects comprising an ozone generator.

The invention seeks to provide an improved apparatus and technique for the sterilisation or disinfection of products, apparatus and instruments, especially dental and surgical tools and instruments.

### PROBLEM TO BE SOLVED BY THE INVENTION

There is a need for improvements in systems, apparatus and methods for sterilization and/or disinfection, especially of dental and surgical tools and instruments.

There is a need for improvements in systems, apparatus and methods for disinfection of wounds, burns and ulcers to improve patient treatment and recovery.

It is an object of this invention to provide a system for use in the disinfection of a wide variety of products, apparatus and instruments and especially dental tools and apparatus.

It is a further object of this invention to provide a system, apparatus and method for use in the disinfection of patient treatment sites such as wounds, ulcers and burns.

### SUMMARY OF THE INVENTION

In accordance with a first aspect of the invention, there is provided an apparatus for the sterilization/disinfecting of dental and/or surgical instruments, the apparatus comprising:
a producing device for producing a disinfecting and/or sterilizing gas composition wherein the gas composition comprises ozone and one or both of hydrogen peroxide and water vapour, the device having a gas outlet for supply of the gas composition;
a treating device for receiving and treating, neutralizing or absorbing a residual gas composition, the device having a gas inlet for receipt of the residual gas composition;
a sterilization/disinfecting chamber in the form of a flexible or collapsible or deformable container, the container having a sealable opening through which a dental/surgical instrument may be placed in the container, and having a container inlet for receiving into the container a disinfecting and/or sterilizing gas composition from the gas outlet of the producing device and a container outlet through which residual gas may be expelled or drawn to the gas inlet of the treating device;
a supply conduit for providing fluid communication from the gas outlet to the container inlet; and
a treating conduit for providing fluid communication from the container outlet to the gas inlet; and
an instrument receptacle for receiving holding or retaining the instrument to be sterilized/disinfected, which receptacle comprises materials and/or moving parts configured to cause the entire surface of the instrument to be sterilized/disinfected to be exposed to the disinfecting and/or sterilizing gas composition, wherein the instrument receptacle comprises
   a tray having a resting surface provided with a plurality of elongate protrusions upon which an elongate instrument may be placed, wherein the elongate protrusions are rollers for manual or automatic rotation, and/or
   a vibrating surface whereby the instruments are caused to move so as to allow the sterilization/disinfecting gas composition to make contact with the entire surface of the instruments being sterilized.

There is provided an apparatus for the sterilization/disinfecting of a product, article or instrument, the apparatus comprising:
a producing device for producing a disinfecting and/or sterilizing fluid composition, the device having a fluid outlet for supply of the fluid composition;
a treating device for receiving and treating, neutralizing or absorbing a residual composition, the device having a fluid inlet for receipt of the residual composition;
a sterilization/disinfecting chamber in the form of a container, the container having a sealable opening through which a dental/surgical instrument may be placed in the container, and having a container inlet for receiving into the container a disinfecting and/or sterilizing fluid composition from the fluid outlet of the producing device and a container outlet through which residual composition may be expelled or drawn to the gas inlet of the treating device;
a supply conduit for providing fluid communication from the fluid outlet to the container inlet; and
a treating conduit for providing fluid communication from the container outlet to the fluid inlet.

The fluid may be a liquid (e.g. an aqueous liquid), but is preferably a gas. Preferably the fluid is an ozone-containing fluid.

In a second aspect of the invention, there is provided a flexible or collapsible or deformable container for use as a sterilization/disinfecting chamber in the system of the first aspect, the container having a sealable opening through which a dental/surgical instrument may be placed in the container, and having a container inlet for receiving into the container a disinfecting and/or sterilizing gas composition from the gas outlet of the producing device and a container outlet through which residual gas may be expelled or drawn to the gas inlet of the treating device, wherein the container further comprises an instrument receptacle for receiving holding or retaining the instrument to be sterilized/disinfected, which receptacle comprises materials and/or moving parts configured to cause the entire surface of the instrument to be sterilized/disinfected to be exposed to the disinfecting and/or sterilizing gas composition.

In a third aspect of the invention, there is provided a method for the sterilization/disinfecting of dental/surgical equipment and tools, the method comprising providing a producing device for producing a disinfecting and/or sterilizing gas composition wherein the gas composition comprises ozone and one or both of hydrogen peroxide and water vapour, the device having a gas outlet for supply of the gas composition, providing a treating device for receiving and treating, neutralizing or absorbing a residual gas composition, the device having a gas inlet for receipt of the expired or excess gas composition, providing a sterilization/disinfecting chamber in the form of a container, the container having a sealable opening through which a dental/surgical instrument may be placed in the container, and having a container inlet for receiving into the container a disinfecting and/or sterilizing gas composition from the gas outlet of the producing device and a container outlet through which residual gas may be expelled or drawn to the gas inlet of the treating device, connecting the gas outlet to the container inlet with a supply conduit for the passage of a gas composition and connecting the container outlet to the gas inlet with a treating conduit for the passage of a gas composition, providing an instrument receptacle for receiving holding or retaining the instrument to be sterilized/disinfected, which receptacle comprises materials and/or moving parts configured to cause the entire surface of the instrument to be sterilized/disinfected to be exposed to the disinfecting and/or sterilizing gas composition, wherein the instrument receptacle comprises a tray having a resting surface provided with a plurality of elongate protrusions upon which an elongate instrument may be placed, wherein the elongate protrusions are rollers for manual or automatic rotation, and/or a vibrating surface whereby the instruments are caused to move so as to allow the sterilization/disinfecting gas composition to make contact with the entire surface of the instruments being sterilized;
placing dental/surgical instruments or tools to be sterilised in the container through the opening and sealing it closed;
causing the producing device to produce the sterilizing/disinfecting gas composition and feeding it to the container so as to place the dental/surgical instruments in contact with the gas composition;
maintaining the presence of gas composition within the chamber for a period of time; and
feeding residual gas composition from the container to the treating device and causing the treating device to treat the residual gas to inhibit release of toxic or environmentally damaging gas to the immediate environment.

There is provided a receptacle for receipt of an article for sterilization, the receptacle comprising materials and/or moving parts configured to cause the entire surface of the article to be sterilized to be exposed to a sterilizing gas composition during a sterilization process.

There is provided an apparatus for the application of a therapeutic or disinfecting fluid to a patient treatment site such as a wound, burn or ulcer, the apparatus comprising:
a producing device for producing a therapeutic or disinfecting fluid composition, the device having a fluid outlet for supply of the fluid composition;
a treating device for receiving and treating, neutralizing or absorbing a residual composition, the device having a fluid inlet for receipt of the residual composition;
a therapeutic or disinfecting chamber having at least one chamber wall and configured for placement in contact with a patient so as to define a treatment space within the chamber between the at least one chamber wall and the patient treatment site, and configured to form an hermetic seal about the patient treatment site and configured with a chamber inlet and a chamber outlet for receiving into the chamber a therapeutic or disinfecting fluid composition from the fluid outlet of the producing device and a chamber outlet through which residual composition may be expelled or drawn to the gas inlet of the treating device;
a supply conduit for providing fluid communication from the fluid outlet to the chamber inlet; and
a treating conduit for providing fluid communication from the chamber outlet to the fluid inlet.

The fluid may be a liquid (e.g. an aqueous liquid), but is preferably a gas. Preferably the fluid is an ozone-containing fluid.

Preferably, the chamber is provided in the form of a patch, bag or envelope, the patch, bag or envelope configured so as to be placed over a patient treatment site so as to define a treatment space between the patch, bag or envelope and the patient treatment site and configured to form a hermetic seal between the patch, bag or envelope and the patient about the treatment space and the patient treatment site.

There is provided a therapeutic or disinfecting chamber, preferably in the form of a patch, bag or envelope, for use in an apparatus as defined above for the application of a therapeutic or disinfecting fluid to a patient treatment site such as a wound, burn or ulcer.

There is provided a method for the application of a therapeutic or disinfecting fluid to a patient treatment site such as a wound, burn or ulcer, the method comprising providing a producing device for producing a therapeutic or disinfecting fluid composition, the device having a fluid outlet for supply of the fluid composition, providing a treating device for receiving and treating, neutralizing or absorbing a residual fluid composition, the device having a fluid inlet for receipt of the expired or excess fluid composition, providing a therapeutic or disinfecting chamber, preferably in the form of a patch, bag or envelope, configured for placement in contact with a patient so as to define a treatment space within the chamber between the at least one chamber wall and the patient treatment site, and configured to form an hermetic seal about the patient treatment site and configured with a chamber inlet and a chamber outlet for receiving into the chamber a therapeutic or disinfecting fluid composition from the fluid outlet of the producing device and a chamber outlet through which residual composition may be expelled or drawn to the gas inlet of the treating device
placing the patch, bag or envelope over the patient treatment site, sealing at least one edge of the patch bag or envelope to the patient to form a hermetic seal;
causing the producing device to produce the therapeutic or disinfecting composition and feeding it to the chamber so as to place the patient treatment site in contact with the fluid composition;
maintaining the presence of fluid composition within the chamber for a period of time; and
optionally feeding residual fluid composition from the chamber to the treating device and causing the treating device to treat the residual fluid to inhibit release of toxic or environmentally damaging fluid to the immediate environment.

There is provided a composition for use in the topical treatment by therapy of a wound, burn or ulcer by providing the composition topically to the wound burn or ulcer in a pre-determined dose for a pre-determined period, the composition comprising a therapeutic gas, preferably ozone.

Preferably, the topical treatment of the composition is provided topically in a pre-determined dose for a pre-determined period by delivering the composition via a dose delivery device comprising a therapeutic or disinfecting chamber according to the seventh aspect above. Preferably, the topical treatment of the composition is provided topically in a pre-determined dose for a predetermined period by generating the composition, delivering the composition to the chamber and removing the composition from the chamber using, for example, an apparatus as per the sixth aspect above.

Preferably, the composition comprises ozone in a concentration of 0.05 to 10% (preferably 0.05 to 5%) in a composition comprising ozone and oxygen. By providing a pre-determined concentration of ozone in a predetermined volume for a predetermined period of contact with the patient site, an accurate therapeutic treatment can be achieved which maximizes therapeutic effect with minimal toxic effect.

### ADVANTAGES OF THE INVENTION

The apparatus, container and method of the invention provides significantly more flexibility and accessibility in the sterilization of products, articles or instruments and in particular dental and surgical instruments. By providing a flexible container, which is optionally disposable, separate from the gas composition producing device, a significantly more portable system is provided. This enables good practice in sterilization of dental and surgical instruments to extend beyond only the larger clinics and thereby reduces the risk of infection through dental and surgical treatment.

The apparatus, chamber, composition and method of the later aspects of the invention provide improved treatment and disinfection of wounds, burns and ulcers. By providing a pre-determined concentration of ozone in a predetermined volume for a predetermined period of contact with the patient site, an accurate therapeutic treatment can be achieved which maximizes therapeutic effect with minimal toxic effect.

### BRIEF DESCRIPTION OF THE DRAWINGS

Figure 1 is a diagrammatic representation of an apparatus of one embodiment of the invention;
Figure 2 is a diagrammatic representation of a container according to one embodiment of the invention;
Figure 3 is a diagrammatic representation of an instrument receptacle according to one embodiment of the invention; and
Figure 4 is a diagrammatic representation of a patch according to an embodiment of another aspect of the invention.

### DETAILED DESCRIPTION OF THE INVENTION

The apparatus, container and method of certain aspects of the present invention is intended for sterilizing and/or disinfecting products, articles or instruments. A wide range of products, articles or instruments may be disinfected and/or sterilized using the apparatus/method such as veterinary apparatus and instruments, surgical apparatus and instruments, dental apparatus and instrument, domestic apparatus and instruments (e.g. for use by children, or in food preparation), commercial food preparation apparatus and instruments, food products, packaged products, food utensils (e.g. knives, forks spoons), etc.

Such products, articles or instruments should simply be capable of withstanding the conditions of the treatment.

Preferably, the apparatus, container and method of the invention is for the sterilization and/or disinfecting of dental or surgical instruments and tools and more preferably dental instruments and tools.

Dental instruments and tools that may be usefully disinfected/sterilized include probes, retractors, mirrors, syringes, drill pieces, burs, excavators, chisels, burnishers, curettes, spatula, forceps, orthodontic tools and orthodontic appliances, endodontic instruments, and other dental apparatus and instruments.

The invention relates to the use of a sterilizing and/or disinfecting gas composition. Hereinafter, the invention will be described with reference to a sterilizing or disinfecting gas composition in general or to ozone in particular but it should be understood that optionally embodiments may also be included in which the term gas/ozone is replaced by fluid, to include ozonated water, where the context allows.

The apparatus comprises a producing device for producing a disinfecting and/or sterilizing gas composition, a treating device for treating, neutralizing or absorbing a residual gas and a sterilization/disinfecting chamber in the form of a container configured for fluid connection with each of the producing device and the treating device. The producing device has a gas outlet, which may be connected by a supply conduit to a container inlet of the container for the supply of a gas composition to the container. The treating device has a gas inlet, which may be connected by a conduit to the container outlet of a container to enable residual gas from the container to be transferred to the treating device for treatment.

The container is a flexible or collapsible or deformable container. For example, the container may be structured and self-supporting container with a collapsible ability, e.g. a cylindrical container with concertinaed sides, or it may be a container not capable of self-supporting. Preferably the container is a bag.

Each of the producing device and the treating device are preferably provided in a housing, preferably the same common housing. Preferably, the container is separate from/external to the housing and is preferably connected by flexible tubing.

The supply and treating conduits preferably comprising flexible tubing conduits configured to connect and provide or enable (e.g. via a controllable valve) fluid communication between the conduit and the producing and treating device(s) (i.e. the gas outlet and gas inlet). The supply and treating conduits are preferably configured, along with the container inlet and outlet to provide a hermetically sealed or sealable fluid communicative connection between the supply conduits and the container. The container may be provided with apertures configured for receiving a suitable configured end of a supply conduit.

Optionally, the supply and treating conduits are provided in a single tube (containing both tubes for example) to a single port in a container.

The container may be of any suitable size or shape depending upon the articles to be disinfected/sterilized. The container should be made of any suitable material, which should preferably not degrade (and should be inert) in the presence of the sterilizing gas composition and residual gas composition at least over the timeframe of a sterilization/disinfection method performed in accordance with the invention. The container should be produced to be robust enough not to readily tear in use, e.g. by provision of thick enough material. Preferably, the container does not contain rubber.

The sterilizing gas composition comprises a sterilizing gas or gas mixture and optionally a quantity non-sterilizing gas or gases.

By sterilizing gas it is meant any gas that is capable of killing microorganisms such as bacteria when the gas is placed in sustained contact with an instrument harbouring such a microorganism. The sterilizing gas comprises a quantity of ozone and one or both of hydrogen peroxide and water vapour. Optionally, the sterilising gas may comprise a peroxide which may be inserted into the container as a peroxide vapour.

Preferably, the gas composition comprises ozone in a concentration of up to about 20%, preferably up to about 10%, more preferably up to about 5%, preferably at least 0.05%, e.g. in the range 0.5 to 2%, preferably 0.75 to 1.5 (e.g. about 1%).

Preferably the ozone has a concentration of at least 1ppm, more preferably at least 50ppm, still more preferably at least 100ppm and yet more preferably at least 200ppm. Optionally, the ozone concentration is 100 to 500 ppm, e.g. 250 to 350ppm. Preferably, the ozone concentration is provided in an amount of up to 100,000 ppm, e.g. up to 50,000 ppm and more preferably in the range 1000 ppm to 20,000 ppm, still more preferably in the range 5000 ppm to 15,000 ppm, e.g. 8,000 to 12,000 ppm.

Optionally ozone is provided in the sterilizing gas composition in an amount of from 1 g/m³, preferably 5 g/m³ to 50 g/m³, preferably 35 g/m³ (e.g. 25-35).

Optionally, the sterilizing gas composition may comprise a quantity of water vapour. In one embodiment, the water vapour derives from the aqueous peroxide solution from which the peroxide gas is generated. Optionally, the sterilizing gas composition may comprise a quantity of air.

Optionally, ozone gas and peroxide vapor may be mixed in the container at the same time.

The sterilizing gas may include activators and promoters for ozone as are known in the art.

By residual gas composition, it is meant gas that is expelled or withdrawn from the container and may include a quantity of sterilizing gas and/or a quantity of partially consumed (e.g. reduced) sterilizing gas.

In one embodiment, the system is configured to purge the container with a purge gas. The container may be purged before supply of the sterilizing gas composition to the container, for example to ensure that the container is hermetically sealed and sterilizing gas (such as ozone) is not going to be released to the local environment from the container during use. Optionally the container may be purged with a purge gas after the sterilizing stage and after the residual sterilizing gas has been expelled/withdrawn, in order to ensure that residual traces of the sterilizing gas are captured. The purge gas is preferably oxygen or may optionally be air. Optionally, the purge gas is a reductant gas, especially for use after the sterilization procedure, in order to ensure no residual ozone remains in the container prior to opening.

Hereinafter the system and method may be described with reference to ozone as a component of the sterilizing gas composition. When reference to ozone is made and the context allows, it should be understood that sterilizing gas composition or another sterilizing gas may be used.

Preferably ozone is generated by the producing device from oxygen in an air supply or oxygen gas from a connected oxygen gas cylinder. The ozone may be generated by any suitable method, e.g. by corona discharge of pure oxygen or oxygen containing gas or by electrochemical generation or by UV ozone generation from oxygen or oxygen containing air. A corona discharge method is preferred. Preferably, the ozone generation is achieved from a cylinder supply of oxygen. Optionally, the ozone generation is achieved from an air supply (e.g. surrounding air) via an inlet to the producing device (and is not produced with an oxygen tank supply).

Residual gas comprising unused ozone is expelled from the container after use and delivered to the treating device (e.g. by suction from the treating device) for treatment. The treatment may comprise neutralization, destruction or absorption of residual ozone. Preferably residual ozone is treated by passing through manganese.

Preferably the producing device and the treating device is a single apparatus achieving both functions. Examples are known, such as the Healozone^{™} apparatus. Accordingly, details of the operation of the Healozone, in particular in connection with the ozone generation and ozone treatment which are useful for the present invention and disclosed at www.healozone.de, such details are incorporated herein by reference.

The system further comprises an instrument receptacle for receiving, holding or retaining an instrument for use in the container. The instrument receptacle may comprise an instrument gripping arrangement or an instrument resting surface. The instrument receptacle may form an integral or removably interconnective part of the container or may be a separate component which may be removed from the container for loading and placed into the container for sterilization of the instruments.

The instrument receptacle may optionally comprise a tray having a resting surface configured for example with a plurality of elongate protrusions, arranged generally in parallel, upon and across which elongate dental and/or surgical instruments may be placed, whereby the majority of the surface of area of the device is not in contact with the resting surface thereby making it readily available for contact by a sterilizing gas.

Optionally, the elongate protrusions are rollers, configured for manual or automatic rotation (e.g. short back and fore rotations) during a sterilization step whereby an instrument resting on the protrusions is caused to move back and forth in a generally longitudinal direction thereby increasing the area of the instrument exposed to sterilizing gas (e.g. the entirety of the surface area).

Optionally, the instrument resting surface of the instrument receptacle, e.g. the protrusions, comprises a gas permeable material, such as an open cell gas permeable foam or membrane material, such that a sterilising gas may permeate through the material to make substantive and sterilizing contact with those parts of the instruments resting on the resting surface (e.g. protrusions).

In an alternative embodiment, the instrument receptacle may be provided with at least one, preferably at least two pairs of gripping elements, each pair of gripping elements comprising two biased together gripping elements capable of receiving and retaining therebetween a part of an instrument. The gripping elements may be rollers operable in the manner described above and optionally may be formed of a gas permeable material.

Optionally, an instrument receptacle comprises a vibrating surface (e.g. battery operated) whereby the instruments are caused to move so as to allow sterilization/disinfecting gas composition to make contact with the entire surface of the articles being sterilized.

Optionally, the system further comprises a support for the container upon which the container may be placed, which support optionally is capable of being vibrated to ensure movement of the articles within the container thereby enabling contact by the sterilizing composition on the entire surface of the articles and, in particular to encourage transport of fluid through any lumens and catheters being sterilized.

Optionally, post sterilization, the container may be disconnected from the devices, whereby the container inlet and outlets may be sealed (e.g. automatically upon removal of the connectors)

According to a preferred embodiment, the container is a bag.

The container may comprise a sealable bag comprising a hermetically sealable opening through which instruments (or an instrument receptacle) may be placed in the bag. The bag may be such that the seal may be opened and closed, or it may be a single use bag (e.g. with a heat seal).

Optionally a bag may be structured, e.g. with ribs, to maintain one, two or three dimensions of the bag in a pre-determined configuration, especially when provided with an inflating gas. Optionally, the bag is provided with a plurality of elongate protrusions on at least one surface, which optionally are formed by rollers.

Optionally the container has a defined structure but is deformable in at least one dimension, e.g. by way of a concertina form of side surfaces of the container whereby it can move from a compact configuration to an extended configuration by purging and extraction of gas, or manually.

The container may be of any suitable size or shape. According to a preferred embodiment in which the container may be used, for example, for sterilization/disinfecting of dental apparatus and instruments, the container may be a bag of at least 100 ml volume, optionally up to 5 litres, preferably in the range 200 ml to 2 litres, more preferably 250 ml to 1 litre, still more preferably 400 to 750 ml.

The bag of a preferred embodiment may be made of any suitable material, such as silicone (e.g. food grade or medical grade silicone) or polypropylene, PEEK, PVF or PVDF or a multi-layer foil bag (e.g. comprising a polyethylene inner layer, aluminum foil and nylon outer layer).

The container is provided with at least one aperture (a connection aperture) for connecting with a conduit to a producing device and a treating device. Optionally a single aperture is provided for both functions (e.g. if the system is configured to provide a single volume of sterilizing gas at any one time, e.g. a bolus of gas), whereby a predetermined volume of sterilizing gas may be provided to the container, where it may be held for a predetermined period and then drawn from the container through the aperture into the treating device (e.g. via a valve between the container and device or within the device). Preferably, two apertures are provided, one for the supply of gas to the container and one for the extraction or expulsion of gas from the container (e.g. enabling a throughflow of gas option).

The apertures are preferably configured so as to provide a hermetic seal when not fitted with connecting conduits to the devices.

The apertures are preferably configured to be interconnective with a conduit, such as flexible tubing, fitted with an adapter or valve on one end. The adaptor or valve may fit into the aperture to enable flow of gas through the aperture to and/or from respective conduits.

The connection apertures of the bag may be provided, for example, with one part of a push-fit connector arrangement (e.g. a female or male part of a male-female push fit connector valve). Alternatively, the connection apertures may be fitted with pierceable septums capable of receiving a supply needle tube provided on the end of a supply conduit. The septum should be configured to form a seal about the supply needle once inserted. The needle in such a connector arrangement should be of sufficient bore to allow the desired flow of sterilising gas composition to and from the container.

The flow of gases to and from the container from the supply and treatment devices may be controlled by a controller according to one or a plurality of pre-set or pre-determined programmes or according to individually selected data, or may be manually controlled (e.g. by actuation of corresponding buttons/switches on a control panel).

Accordingly, the system may be configurable for various lengths of time and various volumes.

The apparatus, chamber, method and composition of certain aspects of the present invention is intended for the application of a therapeutic or disinfecting fluid to a patient treatment site such as a wound, burn or ulcer.

The composition according these aspects is preferably a fluid and more preferably a gaseous composition. Preferably, the composition comprises a therapeutic gas. Any therapeutic gas may be used to provide a desired purpose or effect to the treatment site comprising a wound, burn or ulcer. The therapeutic gas may comprise, for example, a gas (or mixture thereof, where compatible) for topical therapy such as xenon (e.g. for anaesthetic treatment or pain relief at the treatment site), nitric oxide (e.g. for wound healing, or burn healing, e.g. with concomitant systemic anti-inflammatory treatment), oxygen (e.g. for wound healing) and ozone (e.g. for disinfection). Preferably, the composition/fluid comprises ozone.

The gas composition may optionally be provided to comprise ozone in the concentrations set out above in connection with the earlier aspects, but is preferably 0.05 to 10%, more preferably 0.05 to 5%.

The apparatus comprises a producing device for producing a therapeutic or disinfecting fluid composition, a treating device for treating, neutralizing or absorbing a residual gas and a therapeutic or disinfecting chamber configured for fluid connection with each of the producing device and the treating device. The producing device has a fluid outlet, which may be connected by a supply conduit to a chamber inlet of the chamber for the supply of a fluid composition to the chamber. The treating device has a fluid inlet, which may be connected by a conduit to the chamber outlet of a chamber to enable residual fluid from the chamber to be transferred to the treating device for treatment.

Preferably, the chamber is provided in the form of a patch, bag or envelope, the patch, bag or envelope configured so as to be placed over a patient treatment site so as to define a treatment space between the patch, bag or envelope and the patient treatment site and configured to form a hermetic seal between the patch, bag or envelope and the patient about the treatment space and the patient treatment site. The chamber should preferably be external to the producing device and treating device.

Preferably, the patch, bag or envelope is a flexible or collapsible or deformable article. For example, they may be structured and self-supporting with a collapsible ability. The patch, bag or envelope are preferably provided with an adhesive surface for forming an adhesion or otherwise seal against the skin of a patient (or intermediate surface provided on the skin) so as to define when applied a treatment space about the treatment site. Where the chamber is provided by a bag, an opening in the bag provides an opening through which a limb or protrusion of a patient may be placed inside the bag, and edges defining the opening may be provided with adhesion means for forming a seal with the skin and defining the treatment space (e.g. an adhesive composition provided on the edges or an elastic band provided about the exterior surface of the bag). Where the chamber is provided by a patch, the patch may be defined by peripheral portions not intended to overlie the treatment site, a surface of which peripheral portions may be provided with an adhesive composition to secure the patch against the patient's skin and surround the treatment site to provide a hermetic seal. The seal may optionally be formed by an adhesive means (e.g. adhesive composition), a retention means (e.g. elastic band) or resilient border material which forms a seal on the application of pressure.

Insofar as ozone is concerned (and physically insofar as any other therapeutic gas is concerned), the producing and treating devices and tubing conduits may be, where the context allows, as defined in relation to the earlier aspects, any reference to container being a reference to the chamber.

In the present aspects, the chamber may be of any suitable size or shape depending upon the location and size of the treatment site and also on the dose level of therapeutic gas desired.

Optionally a chamber (e.g. patch, bag or envelope) may be structured, e.g. with ribs, to maintain one, two or three dimensions of the chamber in a pre-determined configuration, especially when provided with an inflating gas. Optionally, the chamber (e.g. patch, bag or envelope) is provided with a plurality of elongate protrusions on at least one surface.

Optionally the chamber (e.g. patch, bag or envelope) has a defined structure but is deformable in at least one dimension, e.g. by way of a concertina form of side surfaces of the chamber whereby it can move from a compact configuration to an extended configuration by purging and extraction of gas, or manually.

Optionally, the chamber is provided with a retaining structure to retain the wall of the chamber from contact with the patient between the chamber inlet, patient treatment site and chamber outlet to ensure effective supply of therapeutic gas, contact of the gas with the treatment site and removal of the therapeutic gas residue. Optionally the retaining structure is provided as an integral part of the chamber wall (e.g. an integral part of a patch, bag or envelope, e.g. ribbing or the like) or is provided as a separate insert suitable for placing within the treatment space.

The chamber may be of any suitable size or shape and is preferably sized to cover the treatment site and to provide a desired volume according to the dose required. According to a preferred embodiment the chamber may define at least 100 ml volume, optionally up to 5 litres, preferably in the range 200 ml to 2 litres, more preferably 250 ml to 1 litre, still more preferably 400 to 750 ml.

The patch, bag or envelope (or other chamber wall) should be made of any suitable material, which should preferably not degrade (and should be inert) in the presence of the fluid composition and residual fluid composition at least over the timeframe of a therapeutic method performed in accordance with the invention. It should be produced to be robust enough not to readily tear in use, e.g. by provision of thick enough material. Preferably, the container does not contain rubber. Any suitable material may be used, such as silicone (e.g. food grade or medical grade silicone) or polypropylene, PEEK, PVF or PVDF or a multi-layer foil bag (e.g. comprising a polyethylene inner layer, aluminum foil and nylon outer layer).

The chamber (e.g. patch, bag or envelope) is provided with at least one aperture (a connection aperture) for connecting with a conduit to a producing device and a treating device. Optionally a single aperture is provided for both functions (e.g. if the system is configured to provide a single volume of therapeutic gas at any one time, e.g. a bolus of gas), whereby a predetermined volume of gas may be provided to the chamber, where it may be held for a predetermined period and then drawn from the chamber through the aperture into the treating device (e.g. via a valve between the chamber and device or within the device). Preferably, two apertures are provided, one for the supply of gas to the chamber and one for the extraction or expulsion of gas from the chamber (e.g. enabling a throughflow of gas option).

The apertures are preferably configured so as to provide a hermetic seal when not fitted with connecting conduits to the devices.

The apertures are preferably configured to be interconnective with a conduit, such as flexible tubing, fitted with an adapter or valve on one end. The adaptor or valve may fit into the aperture to enable flow of gas through the aperture to and/or from respective conduits.

The connection apertures of the chamber may be provided, for example, with one part of a push-fit connector arrangement (e.g. a female or male part of a male-female push fit connector valve). Alternatively, the connection apertures may be fitted with pierceable septums capable of receiving a supply needle tube provided on the end of a supply conduit. The septum should be configured to form a seal about the supply needle once inserted. The needle in such a connector arrangement should be of sufficient bore to allow the desired flow of therapeutic gas composition to and from the chamber.

The flow of gases to and from the chamber from the supply and treatment devices may be controlled by a controller according to one or a plurality of pre-set or pre-determined programmes or according to individually selected data, or may be manually controlled (e.g. by actuation of corresponding buttons/switches on a control panel).

Accordingly, the system may be configurable for various lengths of time and various volumes.

By residual gas composition, it is meant gas that is expelled or withdrawn from the container and may include a quantity of sterilizing gas and/or a quantity of partially consumed (e.g. reduced) sterilizing gas.

In one embodiment, the system is configured to purge the chamber with a purge gas. The chamber may be purged before supply of the gas composition to the chamber, for example to ensure that the chamber is hermetically sealed and therapeutic gas (such as ozone) is not going to be released to the local environment from the chamber during use. Optionally the chamber may be purged with a purge gas after the treatment stage and after the residual gas has been expelled/withdrawn, in order to ensure that residual traces of the therapeutic gas are captured. The purge gas is preferably oxygen or may optionally be air.

The ozone production and producing device and residual gas capture and treating device may be as defined above for earlier aspects.

The following relates, unless the context determines otherwise, to all aspects.

Optionally, a sensor may be provided in the container to communicate when the disinfecting gas has achieved a pre-determined extent of disinfection. For example, a visual sensor may be provided which is configured to provide a colour change when a pre-determined level of reaction with ozone (at a rate configured to correspond with the effect of ozone on common microorganisms) has been achieved. Thereby an endpoint in the sterilization can be identified, improving the sterilization performance of the system. Such a sensor may comprise a strip in an ozone-permeable vial, which strip comprises a biological indicator comprising a biological agent and a vital cell indicator which is capable of demonstrating the presence of vital cells (e.g. upon exposure to a certain wavelength fluorescent light).

The ozone producing device and ozone treating device, together forming with the conduits and container/chamber form a closed system for the application and removal and treatment of ozone to a container/chamber as a sterilization/treatment chamber. Together, the ozone and producing and treating devices are formed as a single ozone circulation device.

The ozone circulation device preferably comprises:
- An oxygen supply cylinder/cylinder connector and/or an air inlet
- An air dryer
- A pressure sensor
- An ozone generator
- A moisture trap
- An ozone treatment/neutralizer zone (e.g. manganese bed/filter)
- A vacuum pump
and optionally a hydrogen peroxide solution supply and vapourising chamber, a peroxide scavenging chamber.

The ozone circulation device is preferably portable and preferably lightweight e.g. up to 20 kg, still more preferably up to 12 kg and most preferably up to 10 kg, which more preferably the weights mentioned include the oxygen cylinder.

The ozone circulation device is preferably provided with a controller which is optionally configurable to provide programmes of sterilization protocols. Preferably the method and system of the invention for sterilization/disinfecting of a product, article or instrument is configurable to provide any one or more of the following protocols:
- Protocol 1:
   ∘ Continuous flow:
      ∘ After connection of the supply/treatment conduits to a sealed bag containing an article to be sterilised:
         ▪ Air purge of up to bag volume to bag with outlet closed; hold and pressure sensor (provided in system) determines no leaks
         ▪ Air bolus removed by suction through outlet
         ▪ Continuous supply of humidified ozone gas for period of sterilization
         ▪ Air purge of up to volume of bag followed by suction removal
         ▪ Reductant gas purge of bag followed by suction removal
- Protocol 2
   o Batch sterilization
   ∘ After connection of the supply/treatment conduits to a sealed bag containing an article to be sterilised:
      ▪ Air purge of up to bag volume to bag with outlet closed; hold and pressure sensor (provided in system) determines no leaks
      ▪ Air bolus removed by suction through outlet
      ▪ Bolus of humidified ozone gas for first sterilization period
      ▪ Bolus of ozone gas removed by suction
      ▪ Bolus of humidified ozone gas for second sterilization period
      ▪ Bolus of ozone gas removed by suction
      ▪ Air purge of up to volume of bag followed by suction removal
      ▪ Reductant gas purge of bag followed by suction removal

In each case, other gas compositions may be used in conjunction or in extra steps (e.g. hydrogen peroxide gas, which may be generated by the device or introduced to the bag as a peroxide vapour).

The system is preferably configured to be operated by selection of a programme from one or more options.

The system is preferably configured to be provided in a case, by way of a kit.

The invention will now be described in more detail, without limitation, with reference to the accompanying Figures.

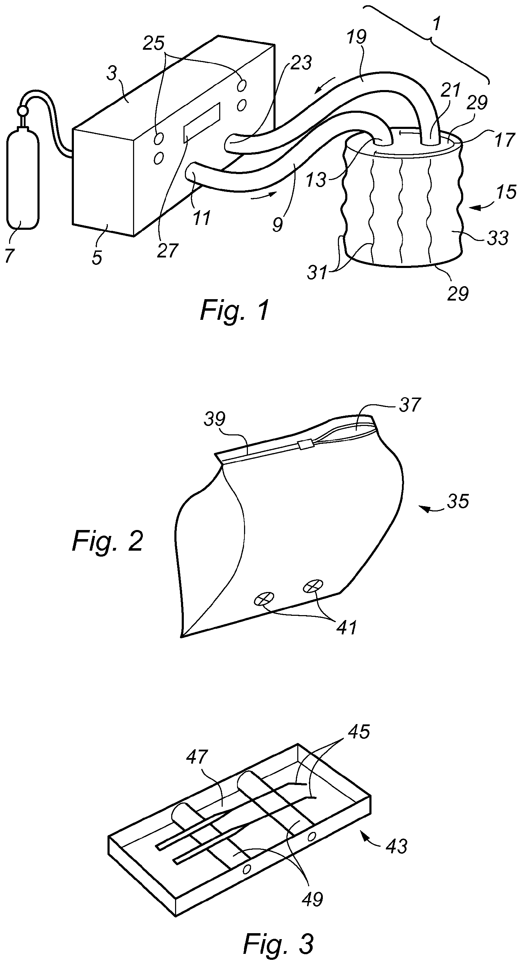

In Figure 1, there is shown a system 1 for sterilising/disinfecting an article such as a dental instrument (not shown) the system 1 comprising an ozone circulation device 3 having a housing 5 containing an ozone generating device and an ozone treatment device, the ozone generating device having an air inlet (not shown) and an oxygen cylinder 7 by way of an oxygen supply. Supply tube 9 of silicone supplies an ozone containing sterilising gas composition from supply outlet 11 of the ozone circulation device 3 to gas inlet 13 of flexible/collapsible sterilisation container 15. Dental instruments may be placed into the container 15 via an hermetically sealed opening 17. Residual gas may be drawn from the container 15 via treatment tube 19 connecting gas outlet 21 of the container 15 to treatment inlet 23 of the treatment device in the housing 5, e.g. by a vacuum. To use the system, a sterilisation programme may be selected via the control switches and display 27 after placing articles in the container 15 and sealing the opening 17. The programme may cause a purge gas into the container followed by a sterilising gas composition comprising ozone along supply tube 9 and into the container 15 for a predetermined time which may then be drawn out through treatment tube 19 followed by a purge/reductant gas to remove residual ozone. The tubes 9,19 may be disconnected from inlet and outlet 13,21 and the sterilised article retrieved.

The container 15 in Figure 1 is one example of a cylindrical container 15 having rigid ends 29 comprising inlet/outlet ports 13,21 and sealable opening 17 in one end 29. Corrugated flexible collapsible rib elements 31 provide structure to a collapsible side 33. Thus on application of a vacuum, the container may optionally contract to distance allowed by the contained article.

In Figure 2, there is illustrated an alternative container 35 in the form of an hermetically sealable bag 35 having a sealable opening 37 with hermetically sealable track system 39 and two inlet/outlet ports 41 for the connection of supply and treatment conduits (not shown) for connection to the ozone generator and residual gas treatment device (not shown).

In Figure 3 is an instrument receptacle 43 for resting dental instruments 45 for sterilisation by placing within a sterilisation chamber (not shown, the receptacle 43 comprising a tray surface 47 having two rotatable cylindrical elements 49 disposed above the tray surface and across the receptacle upon which instruments 45 may be placed. The cylindrical elements 49 preferably comprise ozone permeable surfaces (e.g. open celled foam material) and are rotatable about their axes (e.g. mechanical power, such as wind up) so they are caused to rotate successively back and for so as to cause the instruments 45 to move back and fore along the length of the receptacle 43 during sterilisation so as to expose the entire surfaces of the instruments 45 to the sterilisation gas composition.

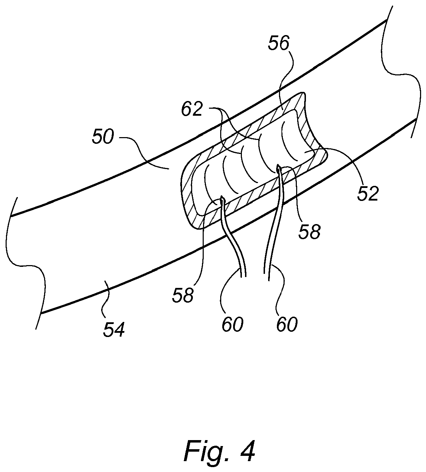

In Figure 4, a patch 50 according to another aspect of the invention is illustrated, the patch comprising a chamber wall 52 defining a chamber and treatment space therein between the patch 50 and a patient site (e.g. harbouring a wound, burn or ulcer) on a patient (in this case a patient's arm) 54. The peripheral border 56 of the patch 50 comprises an adhesive in order to provide a hermetic seal between the patch 50 and the patient's arm 54. The patch wall 54 is provided with two apertures 58 which remain hermetically sealed until fitted with connectors (not shown) of two conduits 60 linking the chamber with a therapeutic gas (typically ozone containing gas) generation and treating device (such as the Healozone^{™}). Therapeutic gas, such as ozone, of a predetermined concentration may be delivered in a predetermined volume into the chamber defined by the patch 50 on the patient's arm via conduits 60 and apertures 58. Ribs 62 may provide some structure to the chamber to enable passage of the therapeutic gas and contact with the treatment site. The gas may be passed over continuously at a predetermined concentration with a pre-determined flow rate for a pre-determined time or a bolus of gas of predetermined concentration and volume may be held in the chamber for a predetermined time to ensure accurate dosage of the gas to the treatment site.

The invention has been described with reference to a preferred embodiment. However, it will be appreciated that variations and modifications can be effected by a person of ordinary skill in the art without departing from the scope of the invention.

## Claims

1. An apparatus for the sterilization/disinfecting of dental and/or surgical instruments, the apparatus comprising:
a producing device (7) for producing a disinfecting and/or sterilizing gas composition wherein the gas composition comprises ozone and one or both of hydrogen peroxide and water vapour, the device having a gas outlet (11) for supply of the gas composition;
a treating device (3) for receiving and treating, neutralizing or absorbing a residual gas composition, the device having a gas inlet (23) for receipt of the residual gas composition;
a sterilization/disinfecting chamber (1) in the form of a flexible or collapsible or deformable container (15, 35), the container having a sealable opening through which a dental/surgical instrument may be placed in the container, and having a container inlet (13) for receiving into the container a disinfecting and/or sterilizing gas composition from the gas outlet (11) of the producing device and a container outlet (29) through which residual gas may be expelled or drawn to the gas inlet (23) of the treating device;
a supply conduit (9) for providing fluid communication from the gas outlet to the container inlet;
a treating conduit (19) for providing fluid communication from the container outlet to the gas inlet; and
an instrument receptacle (43) for receiving holding or retaining the instrument to be sterilized/disinfected, which receptacle comprises materials and/or moving parts configured to cause the entire surface of the instrument to be sterilized/disinfected to be exposed to the disinfecting and/or sterilizing gas composition, wherein the instrument receptacle comprises
a tray (43) having a resting surface (47) provided with a plurality of elongate protrusions (49) upon which an elongate instrument may be placed, wherein the elongate protrusions are rollers for manual or automatic rotation, and/or
a vibrating surface whereby the instruments are caused to move so as to allow the sterilization/disinfecting gas composition to make contact with the entire surface of the instruments being sterilized.

2. An apparatus as claimed in claim 1, wherein the instrument receptacle (43) comprises a tray (43) having a resting surface (47) provided with a plurality of elongate protrusions (49) upon which an elongate instrument may be placed, wherein the elongate protrusions are formed of a gas permeable material.

3. An apparatus as claimed in claim 1 or claim 2, wherein the container (15, 35) comprises a sensor configured to communicate when the disinfecting gas has achieved a pre-determined extent of disinfection.

4. An apparatus as claimed in any one of the preceding claims, wherein the producing device (3) is contained within a housing (5) and the treating device is contained within a housing and wherein the sterilizing/disinfecting chamber (1) container is external to the housing of the producing device and the treating device.

5. An apparatus as claimed in claim 4, wherein a single housing is provided for both the producing device and the treating device.

6. An apparatus as claimed in any one of the preceding claims, wherein the container (35) is a bag.

7. An apparatus as claimed in any one of the preceding claims, wherein the gas composition comprises hydrogen peroxide.

8. An apparatus as claimed in any one of the preceding claims, wherein the gas further comprises water vapour.

9. A flexible or collapsible or deformable container (15, 35) for use as a sterilization/disinfecting chamber (1) in the apparatus of any one of claims 1 to 8, the container having a sealable opening through which a dental/surgical instrument may be placed in the container, and having a container inlet (13) for receiving into the container a disinfecting and/or sterilizing gas composition from the gas outlet (11) of the producing device and a container outlet (29) through which residual gas may be expelled or drawn to the gas inlet (23) of the treating device, wherein the container further comprises an instrument receptacle (43) for receiving holding or retaining the instrument to be sterilized/disinfected, which receptacle comprises materials and/or moving parts configured to cause the entire surface of the instrument to be sterilized/disinfected to be exposed to the disinfecting and/or sterilizing gas composition, wherein the instrument receptacle comprises a tray (43) having a resting surface (47) provided with a plurality of elongate protrusions (49) upon which an elongate instrument may be placed, wherein the elongate protrusions (49) are rollers for manual or automatic rotation and/or a vibrating surface whereby the instruments are caused to move so as to allow the sterilization/disinfecting gas composition to make contact with the entire surface of the articles being sterilized.

10. A method for the sterilization/disinfecting of dental/surgical equipment and tools, the method comprising providing a producing device (3) for producing a disinfecting and/or sterilizing gas composition wherein the gas composition comprises ozone and one or both of hydrogen peroxide and water vapour, the device having a gas outlet for supply of the gas composition, providing a treating device (3) for receiving and treating, neutralizing or absorbing a resisdual gas composition, the device having a gas inlet (23) for receipt of the expired or excess gas composition, providing a sterilization/disinfecting chamber (1) in the form of a container (15, 35), the container having a sealable opening through which a dental/surgical instrument may be placed in the container, and having a container inlet (13) for receiving into the container a disinfecting and/or sterilizing gas composition from the gas outlet (11) of the producing device and a container outlet (29) through which residual gas may be expelled or drawn to the gas inlet of the treating device, connecting the gas outlet to the container inlet with a supply conduit (9) for the passage of a gas composition and connecting the container outlet to the gas inlet with a treating conduit (19) for the passage of a gas composition, providing an instrument receptacle (43) for receiving holding or retaining the instrument to be sterilized/disinfected, which receptacle comprises materials and/or moving parts configured to cause the entire surface of the instrument to be sterilized/disinfected to be exposed to the disinfecting and/or sterilizing gas composition, wherein the instrument receptacle comprises a tray (43) having a resting surface (47) provided with a plurality of elongate protrusions (49) upon which an elongate instrument may be placed, wherein the elongate protrusions are rollers for manual or automatic rotation, and/or a vibrating surface whereby the instruments are caused to move so as to allow the sterilization/disinfecting gas composition to make contact with the entire surface of the instruments being sterilized;
placing dental/surgical instruments or tools to be sterilised in the container through the opening and sealing it closed;
causing the producing device to produce the sterilizing/disinfecting gas composition and feeding it to the container so as to place the dental/surgical instruments in contact with the gas composition;
maintaining the presence of gas composition within the chamber for a period of time;
feeding residual gas composition from the container to the treating device and causing the treating device to treat the residual gas to inhibit release of toxic or environmentally damaging gas to the immediate environment.

11. A method as claimed in claim 10, which comprises a continuous flow protocol or a batch protocol,
wherein a continuous flow protocol comprises, after connection of the supply and treating conduits to a sealed bag containing an article to be sterilised:
▪ an air purge of up to bag volume to bag with outlet closed; hold and determine via a pressure sensor no leaks from the bag;
▪ the air removed by suction through an outlet
▪ a continuous supply of humidified ozone gas for a period of sterilization
▪ an air purge of up to volume of the bag followed by suction removal
▪ a reductant gas purge of the bag followed by suction removal
and wherein a batch protocol comprises, after connection of the supply and treating conduits to a sealed bag containing an article to be sterilised:
▪ an air purge of up to bag volume to bag with outlet closed; hold and determine via a pressure sensor no leaks from the bag
▪ removal of the air bolus by suction through the outlet
▪ provision to the bag of a bolus of humidified ozone gas for a first sterilization period;
▪ removal of the bolus of residual gas by suction
▪ provision to the bag of a bolus of humidified ozone gas for a second sterilization period
▪ removal of the bolus of residual gas by suction
▪ an air purge of up to volume of bag followed by suction removal
▪ a reductant gas purge of bag followed by suction removal.

## Patentansprüche

1. Einrichtung für die Sterilisierung/Desinfektion zahnärztlicher und/oder chirurgischer Instrumente, wobei die Einrichtung Folgendes umfasst:
eine Erzeugungsvorrichtung (7) zum Erzeugen einer desinfizierenden und/oder sterilisierenden Gaszusammensetzung, wobei die Gaszusammensetzung Ozon und eines oder beide von Wasserstoffperoxid und Wasserdampf umfasst, wobei die Vorrichtung einen Gasauslass (11) zum Zuleiten der Gaszusammensetzung aufweist;
eine Behandlungsvorrichtung (3) zum Aufnehmen und Behandeln, Neutralisieren oder Absorbieren einer restlichen Gaszusammensetzung, wobei die Vorrichtung einen Gaseinlass (23) zum Aufnehmen der restlichen Gaszusammensetzung aufweist;
eine Sterilisierungs-/Desinfektionskammer (1) in der Form eines flexiblen oder zusammenklappbaren oder verformbaren Behälters (15, 35), wobei der Behälter eine dicht verschließbare Öffnung aufweist, durch die ein zahnärztliches/chirurgisches Instrument in dem Behälter platziert werden kann, und einen Behältereinlass (13) zum Aufnehmen einer desinfizierenden und/oder sterilisierenden Gaszusammensetzung aus dem Gasauslass (11) der Erzeugungsvorrichtung in dem Behälter und einen Behälterauslass (29), durch den restliches Gas zu dem Gaseinlass (23) der Behandlungsvorrichtung ausgestoßen oder gezogen werden kann, aufweist;
einen Zuleitungskanal (9) zum Vorsehen einer strömungstechnischen Verbindung von dem Gasauslass zu dem Behältereinlass;
einen Behandlungskanal (19) zum Vorsehen einer strömungstechnischen Verbindung von dem Behälterauslass zu dem Gaseinlass; und
eine Instrumentenaufnahme (43) zum Aufnehmen, Halten oder Zurückhalten des zu sterilisierenden/desinfizierenden Instruments, welche Aufnahme Materialien und/oder bewegliche Teile umfasst, die ausgelegt sind, die gesamte Oberfläche des zu sterilisierenden/desinfizierenden Instruments der desinfizierenden und/oder sterilisierenden Gaszusammensetzung auszusetzen, wobei die Instrumentenaufnahme umfasst
eine Schale (43) mit einer Auflagefläche (47), die mit einer Vielzahl länglicher Fortsätze (49) versehen ist, auf welchen ein längliches Instrument platziert werden kann, wobei die länglichen Fortsätze Walzen zur manuellen oder automatischen Drehung sind, und/oder
eine vibrierende Oberfläche, durch die die Instrumente in Bewegung versetzt werden, um so der Sterilisierungs-/Desinfektionsgaszusammensetzung zu ermöglichen, mit der gesamten Oberfläche der zu sterilisierenden Instrumente in Kontakt zu gelangen.

2. Einrichtung nach Anspruch 1, wobei die Instrumentenaufnahme (43) eine Schale (43) mit einer Auflagefläche (47) umfasst, die mit einer Vielzahl länglicher Fortsätze (49) versehen ist, auf welchen ein längliches Instrument platziert werden kann, wobei die länglichen Fortsätze aus einem gasdurchlässigen Material gebildet sind.

3. Einrichtung nach Anspruch 1 oder Anspruch 2, wobei der Behälter (15, 35) einen Sensor umfasst, der eingerichtet ist zu kommunizieren, wann das desinfizierende Gas ein vorab festgelegtes Ausmaß an Desinfektion erreicht hat.

4. Einrichtung nach einem der vorstehenden Ansprüche, wobei die Erzeugungsvorrichtung (3) in einem Gehäuse (5) enthalten ist und die Behandlungsvorrichtung in einem Gehäuse enthalten ist und wobei der Behälter der Sterilisierungs-/Desinfektionskammer (1) außerhalb des Gehäuses der Erzeugungsvorrichtung und der Behandlungsvorrichtung liegt.

5. Einrichtung nach Anspruch 4, wobei ein einziges Gehäuse sowohl für die Erzeugungsvorrichtung als auch die Behandlungsvorrichtung vorgesehen ist.

6. Einrichtung nach einem der vorstehenden Ansprüche, wobei der Behälter (35) ein Beutel ist.

7. Einrichtung nach einem der vorstehenden Ansprüche, wobei die Gaszusammensetzung Wasserstoffperoxid umfasst.

8. Einrichtung nach einem der vorstehenden Ansprüche, wobei das Gas weiter Wasserdampf umfasst.

9. Flexibler oder zusammenklappbarer oder verformbarer Behälter (15, 35) zur Verwendung als eine Sterilisierungs-/Desinfektionskammer (1) in der Einrichtung nach einem der Ansprüche 1 bis 8, wobei der Behälter eine dicht verschließbare Öffnung aufweist, durch die ein zahnärztliches/chirurgisches Instrument in dem Behälter platziert werden kann, und einen Behältereinlass (13) zum Aufnehmen einer desinfizierenden und/oder sterilisierenden Gaszusammensetzung aus dem Gasauslass (11) der Erzeugungsvorrichtung in dem Behälter und einen Behälterauslass (29), durch den restliches Gas zu dem Gaseinlass (23) der Behandlungsvorrichtung ausgestoßen oder gezogen werden kann, aufweist, wobei der Behälter weiter eine Instrumentenaufnahme (43) zum Aufnehmen, Halten oder Zurückhalten des zu sterilisierenden/desinfizierenden Instruments umfasst, welche Aufnahme Materialien und/oder bewegliche Teile umfasst, die ausgelegt sind, die gesamte Oberfläche des zu sterilisierenden/desinfizierenden Instruments der desinfizierenden und/oder sterilisierenden Gaszusammensetzung auszusetzen, wobei die Instrumentenaufnahme eine Schale (43) mit einer Auflagefläche (47), die mit einer Vielzahl länglicher Fortsätze (49) versehen ist, auf welchen ein längliches Instrument platziert werden kann, wobei die länglichen Fortsätze Walzen zur manuellen oder automatischen Drehung sind, und/oder eine vibrierende Oberfläche, durch die die Instrumente in Bewegung versetzt werden, um so der Sterilisierungs-/Desinfektionsgaszusammensetzung zu ermöglichen, mit der gesamten Oberfläche der zu sterilisierenden Artikel in Kontakt zu gelangen, umfasst.

10. Verfahren für die Sterilisierung/Desinfektion zahnärztlicher/chirurgischer Geräte und Werkzeuge, das Verfahren umfassend Vorsehen einer Erzeugungsvorrichtung (3) zum Erzeugen einer desinfizierenden und/oder sterilisierenden Gaszusammensetzung, wobei die Gaszusammensetzung Ozon und eines oder beide von Wasserstoffperoxid und Wasserdampf umfasst, wobei die Vorrichtung einen Gasauslass zum Zuleiten der Gaszusammensetzung aufweist, Vorsehen einer Behandlungsvorrichtung (3) zum Aufnehmen und Behandeln, Neutralisieren oder Absorbieren einer restlichen Gaszusammensetzung, wobei die Vorrichtung einen Gaseinlass (23) zum Aufnehmen der ausgelaufenen oder überschüssigen Gaszusammensetzung aufweist, Vorsehen einer Sterilisierungs-/Desinfektionskammer (1) in der Form eines Behälters (15, 35), wobei der Behälter eine dicht verschließbare Öffnung aufweist, durch die ein zahnärztliches/chirurgisches Instrument in dem Behälter platziert werden kann, und einen Behältereinlass (13) zum Aufnehmen einer desinfizierenden und/oder sterilisierenden Gaszusammensetzung aus dem Gasauslass (11) der Erzeugungsvorrichtung in dem Behälter und einen Behälterauslass (29), durch den restliches Gas zu dem Gaseinlass (23) der Behandlungsvorrichtung ausgestoßen oder gezogen werden kann, aufweist, Verbinden des Gasauslasses mit dem Behältereinlass mit einem Zuleitungskanal (9) für den Durchgang einer Gaszusammensetzung und Verbinden des Behälterauslasses mit dem Gaseinlass mit einem Behandlungskanal (19) für den Durchgang einer Gaszusammensetzung, Vorsehen einer Instrumentenaufnahme (43) zum Aufnehmen, Halten oder Zurückhalten des zu sterilisierenden/desinfizierenden Instruments umfasst, welche Aufnahme Materialien und/oder bewegliche Teile umfasst, die ausgelegt sind, die gesamte Oberfläche des zu sterilisierenden/desinfizierenden Instruments der desinfizierenden und/oder sterilisierenden Gaszusammensetzung auszusetzen, wobei die Instrumentenaufnahme eine Schale (43) mit einer Auflagefläche (47), die mit einer Vielzahl länglicher Fortsätze (49) versehen ist, auf welchen ein längliches Instrument platziert werden kann, wobei die länglichen Fortsätze Walzen zur manuellen oder automatischen Drehung sind, und/oder eine vibrierende Oberfläche, durch die die Instrumente in Bewegung versetzt werden, um so der Sterilisierungs-/Desinfektionsgaszusammensetzung zu ermöglichen, mit der gesamten Oberfläche der zu sterilisierenden Instrumente in Kontakt zu gelangen, umfasst;
Platzieren zahnärztlicher/chirurgischer Instrumente oder Werkzeuge, die zu sterilisieren sind, in dem Behälter durch die Öffnung und dichtes Verschließen derselben;
Veranlasssen, dass die Erzeugungsvorrichtung die sterilisierende/desinfizierende Gaszusammensetzung erzeugt, und Zuleiten derselben zu dem Behälter, so dass die zahnärztlichen/chirurgischen Instrumente in Kontakt mit der Gaszusammensetzung platziert sind;
Aufrechterhalten der Gegenwart einer Gaszusammensetzung in der Kammer über einen Zeitraum;
Zuleiten einer restlichen Gaszusammensetzung aus dem Behälter zu der Behandlungsvorrichtung und Veranlassen, dass die Behandlungsvorrichtung das restliche Gas behandelt, um Freisetzung von toxischem oder umweltschädlichem Gas in die unmittelbare Umgebung zu verhindern.

11. Verfahren nach Anspruch 10, das ein fortlaufendes Ablaufprotokoll oder ein Chargenprotokoll umfasst, wobei ein fortlaufendes Ablaufprotokoll nach Verbindung des Zuleitungs- und Behandlungskanals mit einem dicht verschlossenen Beutel, der einen zu sterilisierenden Artikel enthält, Folgendes umfasst:
- eine Luftspülung bis zum Beutelvolumen an dem Beutel mit geschlossenem Auslass; Halten und Bestimmen, über einen Drucksensor, dass der Beutel nicht leckt;
- Absaugen der Luft durch einen Auslass
- eine fortlaufende Zuleitung von befeuchtetem Ozongas während einer Sterilisationsperiode
- eine Luftspülung bis zum Beutelvolumen, gefolgt von Absaugen
- eine Reduktionsmittelgasspülung des Beutels, gefolgt von Absaugen
und wobei ein Chargenprotokoll, nach Verbindung des Zuleitungs- und Behandlungskanals mit einem dicht verschlossenen Beutel, der einen zu sterilisierenden Artikel enthält, Folgendes umfasst:
- eine Luftspülung bis zum Beutelvolumen an dem Beutel mit geschlossenem Auslass; Halten und Bestimmen, über einen Drucksensor, dass der Beutel nicht leckt
- Entfernen des Luftbolus durch Absaugen durch den Auslass
- Versehen des Beutels mit einem Bolus befeuchteten Ozongases für eine erste Sterilisationsperiode;
- Entfernen des Bolus von restlichem Gas durch Absaugen
- Versehen des Beutels mit einem Bolus befeuchteten Ozongases für eine zweite Sterilisationsperiode
- Entfernen des Bolus von restlichem Gas durch Absaugen
- eine Luftspülung bis zum Beutelvolumen, gefolgt von Absaugen
- eine Reduktionsmittelgasspülung des Beutels, gefolgt von Absaugen.

## Revendications

1. Appareil pour la stérilisation/désinfection d'instruments dentaires et/ou chirurgicaux, l'appareil comprenant :
un dispositif de production (7) pour produire une composition de gaz de désinfection et/ou de stérilisation, dans lequel la composition de gaz comprend de l'ozone et l'un, ou l'un et l'autre, du peroxyde d'hydrogène et de la vapeur d'eau, le dispositif présentant une sortie de gaz (11) pour la fourniture de la composition de gaz ;
un dispositif de traitement (3) pour recevoir et traiter, neutraliser ou absorber une composition de gaz résiduel, le dispositif présentant une entrée de gaz (23) pour la réception de la composition de gaz résiduel ;
une chambre de stérilisation/désinfection (1) sous la forme d'un récipient souple ou pliable ou déformable (15, 35), le récipient présentant une ouverture pouvant être scellée par laquelle un instrument dentaire/chirurgical peut être placé dans le récipient, et présentant une entrée de récipient (13) pour recevoir, dans le récipient, une composition de gaz de désinfection et/ou de stérilisation en provenance de la sortie de gaz (11) du dispositif de production, et une sortie de récipient (29) par laquelle un gaz résiduel peut être expulsé ou aspiré vers l'entrée de gaz (23) du dispositif de traitement ;
un conduit d'alimentation (9) pour établir une communication fluidique à partir de la sortie de gaz jusqu'à l'entrée de récipient ;
un conduit de traitement (19) pour fournir une communication fluidique à partir de la sortie de récipient jusqu'à l'entrée de gaz ; et
un réceptacle d'instrument (43) pour recevoir, supporter ou retenir l'instrument à stériliser/désinfecter, lequel réceptacle comprend des matériaux et/ou des pièces mobiles configurées pour amener toute la surface de l'instrument qui doit être stérilisé/désinfecté, à être exposée à la composition de gaz de désinfection et/ou de stérilisation, dans lequel le réceptacle d'instrument comprend
un plateau (43) présentant une surface d'appui (47) pourvue d'une pluralité de saillies allongées (49) sur laquelle un instrument allongé peut être placé, dans lequel les saillies allongées sont des rouleaux pour une rotation manuelle ou automatique et/ou
une surface vibrante au moyen de laquelle les instruments sont amenés à se déplacer de sorte à permettre à la composition de gaz de stérilisation/désinfection de réaliser un contact avec toute la surface des instruments qui sont stérilisés.

2. Appareil selon la revendication 1, dans lequel le réceptacle d'instrument (43) comprend un plateau (43) présentant une surface d'appui (47) pourvue d'une pluralité de saillies allongées (49) sur laquelle un instrument allongé peut être placé, dans lequel les saillies allongées sont formées d'un matériau perméable aux gaz.

3. Appareil selon la revendication 1 ou la revendication 2, dans lequel le récipient (15, 35) comprend un capteur configuré pour communiquer lorsque le gaz de désinfection a atteint un degré prédéterminé de désinfection.

4. Appareil selon l'une quelconque des revendications précédentes, dans lequel le dispositif de production (3) est contenu à l'intérieur d'un boîtier (5) et le dispositif de traitement est contenu à l'intérieur d'un boîtier et dans lequel le récipient de chambre de stérilisation/désinfection (1) est externe au boîtier du dispositif de production et du dispositif de traitement.

5. Appareil selon la revendication 4, dans lequel un seul boîtier est prévu à la fois pour le dispositif de production et le dispositif de traitement.

6. Appareil selon l'une quelconque des revendications précédentes, dans lequel le récipient (35) est un sac.

7. Appareil selon l'une quelconque des revendications précédentes, dans lequel la composition de gaz comprend du peroxyde d'hydrogène.

8. Appareil selon l'une quelconque des revendications précédentes, dans lequel le gaz comprend en outre de la vapeur d'eau.

9. Récipient souple ou pliable ou déformable (15, 35) destiné à être utilisé comme chambre de stérilisation/désinfection (1) dans l'appareil selon l'une quelconque des revendications 1 à 8, le récipient présentant une ouverture pouvant être scellée par laquelle un instrument dentaire/chirurgical peut être placé dans le récipient, et présentant une entrée de récipient (13) pour recevoir, dans le récipient, une composition de gaz de désinfection et/ou de stérilisation en provenance de la sortie de gaz (11) du dispositif de production, et une sortie de récipient (29) par laquelle un gaz résiduel peut être expulsé ou aspiré vers l'entrée de gaz (23) du dispositif de traitement, dans lequel le récipient comprend en outre un réceptacle d'instrument (43) pour recevoir, supporter ou retenir l'instrument à stériliser/désinfecter, lequel réceptacle comprend des matériaux et/ou des pièces mobiles configurées pour amener toute la surface de l'instrument qui doit être stérilisé/désinfecté, à être exposée à la composition de gaz de désinfection et/ou de stérilisation, dans lequel le réceptacle d'instrument comprend un plateau (43) présentant une surface d'appui (47) pourvue d'une pluralité de saillies allongées (49) sur laquelle un instrument allongé peut être placé, dans lequel les saillies allongées (49) sont des rouleaux pour une rotation manuelle ou automatique et/ou une surface vibrante au moyen de laquelle les instruments sont amenés à se déplacer de sorte à permettre à la composition de gaz de stérilisation/désinfection de réaliser un contact avec toute la surface des instruments qui sont stérilisés.

10. Procédé pour la stérilisation/désinfection d'un équipement dentaire/chirurgical et d'outils dentaires/chirurgicaux, le procédé comprenant la fourniture d'un dispositif de production (3) pour produire une composition de gaz de désinfection et/ou de stérilisation, dans lequel la composition de gaz comprend de l'ozone et l'un, ou l'un et l'autre, du peroxyde d'hydrogène et de la vapeur d'eau, le dispositif présentant une sortie de gaz pour la fourniture de la composition de gaz, pour fournir un dispositif de traitement (3) pour recevoir et traiter, neutraliser ou absorber une composition de gaz résiduel, le dispositif présentant une entrée de gaz (23) pour la réception de la composition de gaz résiduel, la fourniture d'une chambre de stérilisation/désinfection (1) sous la forme d'un récipient (15, 35), le récipient présentant une ouverture pouvant être scellée par laquelle un instrument dentaire/chirurgical peut être placé dans le récipient, et présentant une entrée de récipient (13) pour recevoir, dans le récipient, une composition de gaz de désinfection et/ou de stérilisation en provenance de la sortie de gaz (11) du dispositif de production, et une sortie de récipient (29) par laquelle un gaz résiduel peut être expulsé ou aspiré vers l'entrée de gaz du dispositif de traitement, le raccordement de la sortie de gaz à l'entrée de récipient avec un conduit d'alimentation (9) pour le passage d'une composition de gaz et le raccordement de la sortie de récipient à l'entrée de gaz avec un conduit de traitement (19) pour le passage d'une composition de gaz, la fourniture d'un réceptacle d'instrument (43) pour recevoir, supporter ou retenir l'instrument à stériliser/désinfecter, lequel réceptacle comprend des matériaux et/ou des pièces mobiles configurées pour amener toute la surface de l'instrument qui doit être stérilisé/désinfecté, à être exposée à la composition de gaz de désinfection et/ou de stérilisation, dans lequel le réceptacle d'instrument comprend un plateau (43) présentant une surface d'appui (47) pourvue d'une pluralité de saillies allongées (49) sur laquelle un instrument allongé peut être placé, dans lequel les saillies allongées sont des rouleaux pour une rotation manuelle ou automatique et/ou une surface vibrante au moyen de laquelle les instruments sont amenés à se déplacer de sorte à permettre à la composition de gaz de stérilisation/désinfection de réaliser un contact avec toute la surface des instruments qui sont stérilisés ;
le placement d'instruments, ou d'outils, dentaires/chirurgicaux à stériliser dans le récipient par l'ouverture et le scellement de celle-ci fermée ;
la provocation du dispositif de production à produire la composition de gaz de stérilisation/désinfection et l'introduction de celle-ci dans le récipient de sorte à placer les instruments dentaires/chirurgicaux en contact avec la composition de gaz ;
le maintien de la présence d'une composition de gaz à l'intérieur de la chambre pendant une période de temps ;
l'introduction de la composition de gaz résiduel provenant du récipient dans le dispositif de traitement et la provocation du dispositif de traitement pour traiter le gaz résiduel pour empêcher la libération d'un gaz toxique ou nocif pour l'environnement dans l'environnement immédiat.

11. Procédé selon la revendication 10, qui comprend un protocole en flux continu ou un protocole par lots, dans lequel un protocole en flux continu comprend, après le raccordement des conduits d'alimentation et de traitement à un sac scellé contenant un article à stériliser :
- une purge d'air jusqu'à un volume de sac pour un sac présentant une sortie fermée ; le maintien et la détermination, par le biais d'un capteur de pression, qu'il n'y a pas de fuite du sac ;
- l'air éliminé par aspiration par une sortie
- une alimentation continue en ozone gazeux humidifié pendant une période de stérilisation
- une purge d'air jusqu'à un volume du sac suivie par une élimination par aspiration
- une purge de gaz réducteur du sac suivie par une élimination par aspiration
et dans lequel un protocole par lots comprend, après le raccordement des conduits d'alimentation et de traitement à un sac scellé contenant un article à stériliser :
- une purge d'air jusqu'à un volume de sac pour un sac présentant une sortie fermée ; le maintien et la détermination, par le biais d'un capteur de pression, qu'il n'y a pas de fuite du sac
- l'élimination du bolus d'air par aspiration par la sortie
- la fourniture au sac d'un bolus d'ozone gazeux humidifié pendant une première période de stérilisation ;
- l'élimination du bolus de gaz résiduel par aspiration
- la fourniture au sac d'un bolus d'ozone gazeux humidifié pendant une seconde période de stérilisation
- l'élimination du bolus de gaz résiduel par aspiration
- une purge d'air jusqu'à un volume du sac suivie par une élimination par aspiration
- une purge de gaz réducteur du sac suivie par une élimination par aspiration.
